# EUROPEAN PATENT SPECIFICATION

(11) **EP 2 308 991 B1**
(45) Date of publication and mention of the grant of the patent: **03.07.2013**
(21) Application number: 10251732.3
(22) Date of filing: 04.10.2010
(51) Int. Cl.: C12Q 1/00, G01N 27/00, G01N 33/52

(54) **Multi-analyte test strip with inline working electrodes and shared opposing counter/reference electrode**
Teststreifen für mehrere Analyte mit gemeinsam genutzter Gegen-/Referenzelektrode und reihenförmige Elektrodenkonfiguration
Bande de test multi-analytes dotée d'une contre-électrode/électrode de référence partagée et configuration d'électrodes en ligne

(30) Priority: 02.10.2009 US 572534
(43) Date of publication of application: 13.04.2011
(73) Proprietor: Lifescan Scotland Limited, Inverness-shire IV2 3ED (GB)
(72) Inventor: Webster, Graeme, Inverness, Inverness-shire IV2 4GS (GB); Cardosi, Marco F, Inverness-shire IV2 5FP (GB); Saini, Selwayan, Dingwall IV7 8JS (GB)
(74) Representative: Tunstall, Christopher Stephen

(56) References cited:
- EP-A1- 2 292 785
- WO-A2-02/06806
- WARD W KENNETH ET AL: "A wire-based dual-analyte sensor for glucose and lactate: in vitro and in vivo evaluation.", DIABETES TECHNOLOGY & THERAPEUTICS JUN 2004 LNKD- PUBMED:15198844, vol. 6, no. 3, June 2004 (2004-06), pages 389-401, XP002611374, ISSN: 1520-9156
- FORROW N J ET AL: "Development of a commercial amperometric biosensor electrode for the ketone d-3-hydroxybutyrate", BIOSENSORS AND BIOELECTRONICS, ELSEVIER BV, NL, vol. 20, no. 8, 15 February 2005 (2005-02-15), pages 1617-1625, XP004697382, ISSN: 0956-5663, DOI: DOI:10.1016/J.BIOS.2004.07.009
- WANG J: "Portable electrochemical systems", TRAC, TRENDS IN ANALYTICAL CHEMISTRY, ELSEVIER, AMSTERDAM, NL, vol. 21, no. 4, 1 April 2002 (2002-04-01), pages 226-232, XP004371240, ISSN: 0165-9936, DOI: DOI:10.1016/S0165-9936(02)00402-8
- D'ORAZIO PAUL: "Biosensors in clinical chemistry.", CLINICA CHIMICA ACTA; INTERNATIONAL JOURNAL OF CLINICAL CHEMISTRY AUG 2003 LNKD- PUBMED:12867275, vol. 334, no. 1-2, August 2003 (2003-08), pages 41-69, XP002611375, ISSN: 0009-8981
- WILSON G S ET AL: "Biosensors for real-time in vivo measurements", BIOSENSORS AND BIOELECTRONICS, ELSEVIER BV, NL, vol. 20, no. 12, 15 June 2005 (2005-06-15) , pages 2388-2403, XP004861155, ISSN: 0956-5663, DOI: DOI:10.1016/J.BIOS.2004.12.003
- YOUN WANG, HUI XU, JIANMING ZHANG, GUANG LI: "Electrochemical Sensors for Clinic Analysis", SENSORS, vol. 8, 27 March 2008 (2008-03-27), pages 2043-2081, XP002611376, ISSN: 1424-8220
- LI G ET AL: "A new handheld biosensor for monitoring blood ketones", SENSORS AND ACTUATORS B, ELSEVIER SEQUOIA S.A., LAUSANNE, CH, vol. 109, no. 2, 14 September 2005 (2005-09-14), pages 285-290, XP025328964, ISSN: 0925-4005, DOI: DOI:10.1016/J.SNB.2004.12.060 [retrieved on 2005-09-14]

## Description

### BACKGROUND OF THE INVENTION

Field of the Invention

The present invention relates, in general, to medical devices and, in particular, to analyte test strips, test meters and related methods.

Description of Related Art

The determination (e.g., detection and/or concentration measurement) of an analyte in a fluid sample is of particular interest in the medical field. For example, it can be desirable to determine glucose, ketones, cholesterol, acetaminophen and/or HbA1c concentrations in a sample of a bodily fluid such as urine, blood or interstitial fluid. Such determinations can be achieved using analyte test strips, based on, for example, photometric or electrochemical techniques, along with an associated test meter.

Typical electrochemical-based analyte test strips employ a working electrode along with an associated counter/reference electrode and enzymatic reagent to facilitate an electrochemical reaction with a single analyte of interest and, thereby, determine the concentration of that single analyte. For example, an electrochemical-based analyte test strip for the determination of glucose concentration in a blood sample can employ an enzymatic reagent that includes the enzyme glucose oxidase and the mediator ferricyanide. Such conventional analyte test strips are described in, for example, U.S. Patent No.s 5,708,247; 5,951,836; 6,241,862; and 6,284,125.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified perspective, exploded depiction of a co-facial multi-analyte test strip according to an embodiment of the present invention;
FIGs. 2A-2F are simplified top views of the first insulating layer, electrically conductive layer, multi-analyte reagent layer, patterned spacer layer, shared counter/reference electrode layer and second insulating layer, respectively, of the co-facial multi-analyte test strip of FIG. 1;
FIG. 3 is a simplified top view of the co-facial multi-analyte test strip of FIG. 1;
FIG. 4 is simplified depiction of the electrically conductive layer and shared counter/reference electrode layer of a co-facial multi-analyte test strip according to an embodiment of the present invention in use with a test meter also according to an embodiment of the present invention; and
FIG. 5 is a flow diagram depicting stages in a process for determining multiple analytes in a single bodily fluid sample according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Co-facial (also sometimes referred to as "opposing electrode", "opposing" or "opposed") multi-analyte test strips according to embodiments of the present invention include a first insulating layer with an electrically conductive layer disposed thereon. The electrically conductive layer includes a first working electrode with a first analyte contact pad and a second working electrode with a second analyte contact pad. In addition, the first working electrode and the second working electrode of the electrically conductive layer are disposed on the first insulating layer in a planar inline configuration.

The co-facial multi-analyte test strips also include a patterned spacer layer positioned above the electrically conductive layer, with the patterned spacer layer defining a single bodily fluid (e.g., whole blood) sample-receiving chamber therein that overlies the first working electrode and the second working electrode. The multi-analyte test strips further include a shared counter/ reference electrode layer overlying and exposed to the single bodily-fluid sample receiving chamber and configured in an opposing (i.e., co-facial) relationship to the first and second working electrodes. The shared counter/reference electrode has a counter/reference electrode contact pad.

The co-facial multi-analyte test strips also have a second insulating layer disposed above the shared counter/reference electrode layer. Moreover, the co-facial multi-analyte test strips have a multi-analyte reagent layer disposed on the electrically conductive layer with the multi-analyte reagent layer having a first analyte reagent portion (such as a glucose reagent portion) disposed on the first working electrode within the bodily fluid sample-receiving chamber and a second analyte reagent portion (e.g., a ketone reagent portion) disposed the second working electrode within the bodily fluid sample receiving chamber.

Co-facial multi-analyte test strips according to the present invention are beneficial in that a plurality of non-identical analytes (e.g., the analyte glucose and the ketone analyte 3-hydroxybutyrate) can be determined in a single bodily fluid sample (such as a single whole blood sample). In addition, since the co-facial multi-analyte test strips include a shared counter/reference electrode layer (i.e., a counter/reference electrode that is used during the determination of both the first analyte and the second analyte), the co-facial multi-analyte test strips, and their bodily fluid sample-receiving chamber, are beneficially small in size. Moreover, the use of an inline configuration for the first working electrode and second working electrode enables the co-facial multi-analyte test strips to employ a straight sample chamber with straight bodily fluid sample flow, thus simplifying manufacturing and operation of the co-facial multi-analyte test strip. Furthermore, since the shared reference/counter electrode layer is configured in an opposing (i.e., co-facial) configuration with respect to the first working electrode and the second working electrode, the sample-receiving chamber has a beneficially small volume and the overall test strip can be beneficially compact.

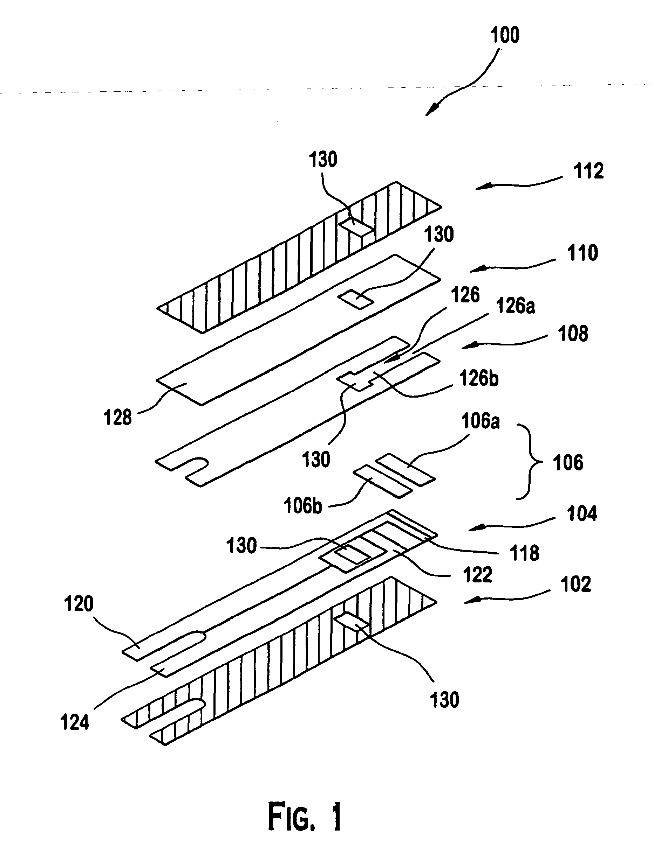

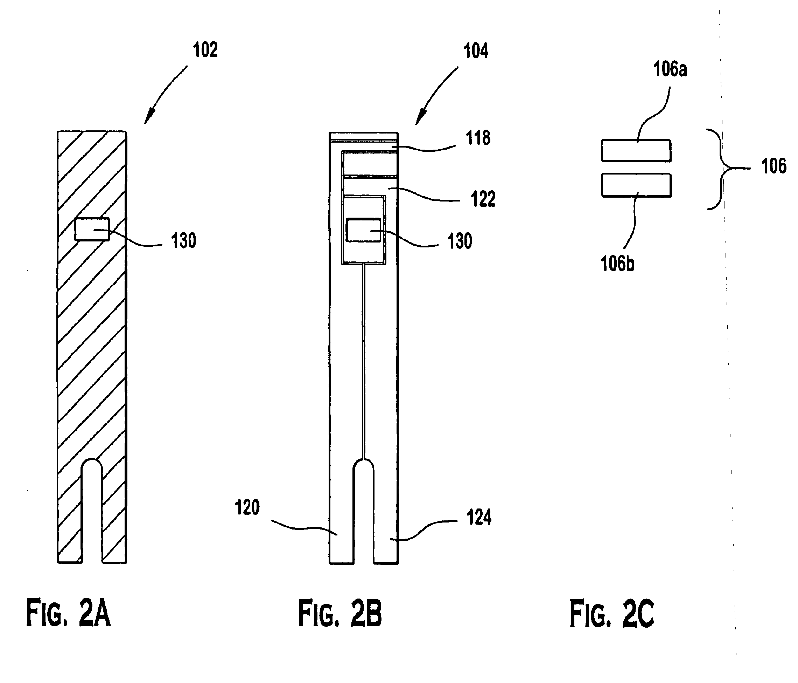

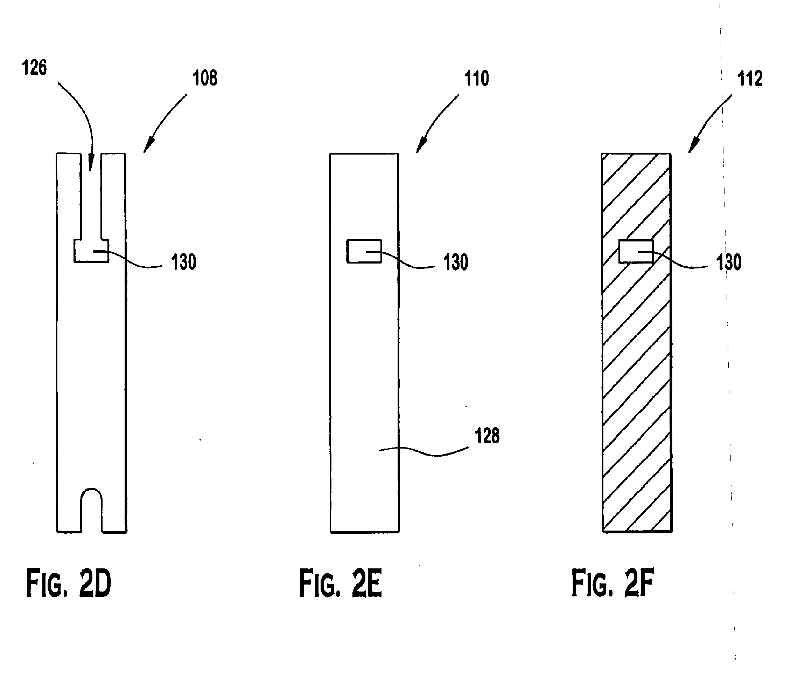

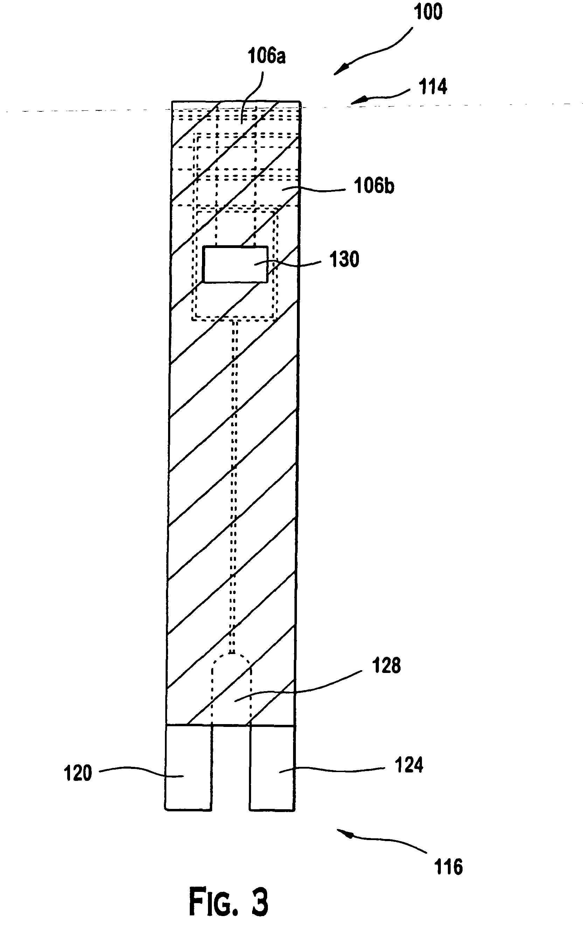

FIG. 1 is a simplified perspective, exploded depiction of a co-facial multi-analyte test strip 100 according to an embodiment of the present invention. FIGs. 2A-2F are simplified top views of a first insulating layer 102, electrically conductive layer 104, multi-analyte reagent layer 106, patterned spacer layer 108, shared counter/reference electrode layer 110 and second insulating layer 112, respectively, of co-facial multi-analyte test strip 100. FIG. 3 is a simplified top view of co-facial multi-analyte test strip 100.

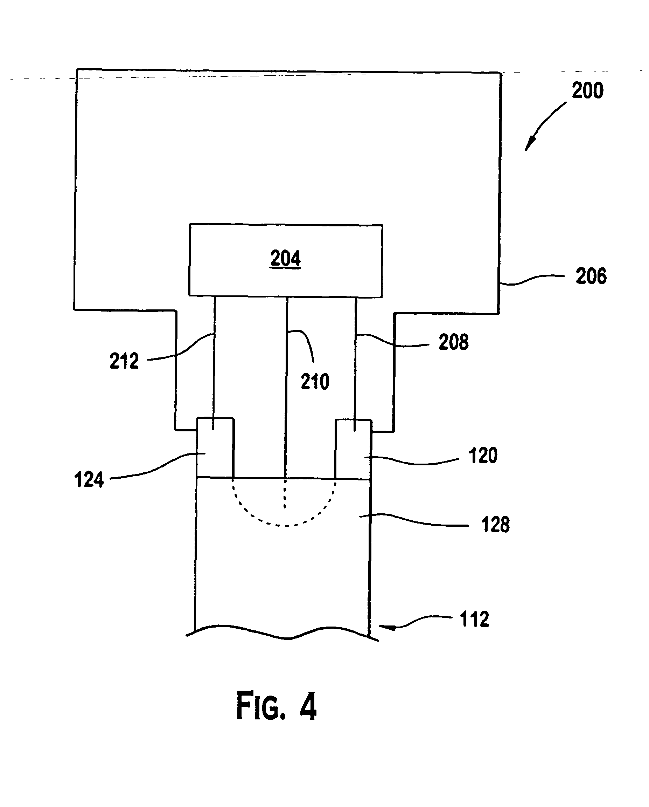

Referring to FIG. 1, FIGs. 2A through 2F and FIG. 3, co-facial multi-analyte test strip 100 is configured for use with a test meter (described further herein, for example with respect to the embodiment of FIG. 4) and includes a proximal end 114 and a distal end 116 (see FIG. 3). Co-facial multi-analyte test strip 100 also includes first insulating layer 102, with electrically conductive layer 104 disposed thereon.

Electrically conductive layer 104 includes a first working electrode 118 with a first analyte contact pad 120 and a second working electrode 122 with a second analyte contact pad 124 (see Fig. 2B in particular). Patterned spacer layer 108 of co-facial multi-analyte test strip 100 is disposed above electrically conductive layer 104 (see FIG. 1 in particular), with the patterned spacer layer defining a single bodily fluid (e.g., whole blood) sample-receiving chamber 126 therein that overlies first working electrode 118 and second working electrode 122. In addition, single bodily fluid sample-receiving chamber 126 has a proximal end 126a and a distal end 126b.

Shared counter/ reference electrode layer 110 overlies, and is exposed to, single bodily fluid sample-receiving chamber 126 and is configured in an opposing relationship to first working electrode 118 and second working electrode 122. In addition, shared counter/reference electrode has a counter/reference electrode contact pad 128.

Second insulating layer 112 of co-facial multi-analyte test strip 100 is disposed above shared counter/reference electrode layer 110, as depicted in FIG. 1.

Co-facial multi-analyte test strip 100 includes multi-analyte reagent layer 106 disposed on electrically conductive layer 104 (see FIG. 1 in particular). Multi-analyte reagent layer 106 has a first analyte reagent portion 106a (such as a glucose reagent portion) disposed on first working electrode 118 within single bodily fluid sample-receiving chamber 126 and a second analyte reagent portion 106b (e.g., a ketone reagent portion) disposed on second working electrode 122 within single bodily fluid sample-receiving chamber 126.

In the embodiment of FIGs. 1, 2A-2F and 3, first analyte contact pad 120, counter/reference electrode contact pad 128, and second analyte contact pad 124 are configured for contact with electrical connector pins of a test meter. Moreover, the first working electrode 118 and second working electrode 122 are disposed on first insulating layer 102 in a planar inline configuration beneath single bodily fluid sample-receiving chamber 126. Therefore, a bodily fluid sample applied to proximal end 126a of single bodily fluid sample-receiving chamber 126 will be conducted down the single bodily fluid sample-receiving chamber 126 and across first working electrode 118 and subsequently across second working electrode 120 since the first and second working electrodes are in-line with one another with respect to the direction of bodily fluid sample flow.

Co-facial multi-analyte test strip 100 also includes a vent opening 130 that extends entirely through first insulating layer 102, electrically conductive layer 104, patterned spacer layer 108, shared counter/reference electrode layer 110 and second insulating layer 112. Vent opening 130 is in fluidic communication with distal end 126b of single bodily fluid sample-receiving chamber 126 and facilitates the conduction of a bodily fluid sample down the single bodily fluid sample-receiving chamber 126 by capillary forces. Moreover, vent opening 130 is further configured as a stop flow junction at distal end 126b of the single bodily fluid sample-receiving chamber 126. Such a stop flow junction is configured to prevent a bodily fluid sample from exiting distal end 126b of single bodily fluid sample-receiving chamber 126 due to an abrupt change in fluid flow cross-section at the intersection of vent opening 130 and distal end 126b.

The manufacturing of co-facial multi-analyte test strip 100 is beneficially simplified by having vent opening 130 extend entirely through co-facial multi-analyte test strip 100. For example, vent opening 130 can be created using a conventional and simple punch processing after first insulating layer 102, electrically conductive layer 104, patterned spacer layer 108, shared counter/reference electrode layer 110 and second insulating layer 112 have been assembled. Such punch processing also eliminates the need to align individual vent openings created in each of the first insulating layer 102, electrically conductive layer 104, patterned spacer layer 108, shared counter/reference electrode layer 110 and second insulating layer 112. In addition, such a vent opening is open to the test strip surroundings at the outer surface of both the first and second insulating layers, thus increasing vent reliability via redundancy. However, once apprised of this disclosure, one skilled in the art will recognize that co-facial multi-analyte test strips according to embodiments of this invention can include a vent opening that provides fluidic communication between distal end 126 and the external surface of either first insulating layer 102 or second insulating layer 112.

First insulating layer 102 and second insulating layer 112 can be formed, for example, of a suitable plastic (e.g., PET, PETG, polyimide, polycarbonate, polystyrene), silicon, ceramic, or glass material. For example, the first and second insulating layers can be formed from a 7 mil polyester substrate.

In the embodiment of FIGs. 1, 2A-2F and 3, first working electrode 118 and shared counter/reference electrode layer 110, along first analyte reagent portion 106a, are configured to electrochemically determine a first analyte concentration in a bodily fluid sample (such as glucose in a whole blood sample) using any suitable electrochemical-based technique known to one skilled in the art. Furthermore, second working electrode 122 and shared counter/reference electrode layer 110, along with second analyte reagent portion 106b, are configured to electrochemically determine a second analyte concentration in the same bodily fluid sample (such as the ketone 3-hydroxybutyrate).

First electrically conductive layer 104 can be formed of any suitable conductive material such as, for example, gold, palladium, carbon, silver, platinum, tin oxide, iridium, indium, or combinations thereof (e.g., indium doped tin oxide). Moreover, any suitable technique can be employed to form first electrically conductive layer 104 including, for example, sputtering, evaporation, electro-less plating, screen-printing, contact printing, or gravure printing. For example, first electrically conductive layer 104 can be a sputtered palladium layer with the first and second working electrodes defined via laser ablation.

Shared counter/reference electrode layer 110 can, for example, be a gold layer that is sputter coated in the underside of second insulating layer 112 using conventional techniques known in the art. In addition, vent opening 130 can be formed using conventional punching techniques and punch tooling.

Patterned spacer layer 108 serves to bind together first insulating layer 102 (with first electrically conductive layer 104 thereon) and second insulating layer 112 with shared counter/reference electrode layer 110 on the underside thereof. Patterned spacer layer 108 can be, for example, a 95um thick, double-sided pressure sensitive adhesive layer, a heat activated adhesive layer, or a thermo-setting adhesive plastic layer. Patterned spacer layer 108 can have, for example, a thickness in the range of from about 1 micron to about 500 microns, preferably between about 10 microns and about 400 microns, and more preferably between about 40 microns and about 200 microns.

First analyte reagent portion 106a can be any suitable mixture of reagents known to those of skill in the art that selectively reacts with a first analyte, such as, for example glucose, in a bodily fluid sample to form an electroactive species, which can then be quantitatively measured at the first working electrode of co-facial multi-analyte test strips according to embodiments of the present invention. Therefore, first analyte reagent portion 106b includes at least an enzyme and a mediator. Examples of suitable mediators include, for example, ferricyanide, ferrocene, ferrocene derivatives, osmium bipyridyl complexes, and quinone derivatives. Examples of suitable enzymes include glucose oxidase, glucose dehydrogenase (GDH) using a pyrroloquinoline quinone (PQQ) co-factor, GDH using a nicotinamide adenine dinucleotide (NAD) co-factor, and GDH using a flavin adenine dinucleotide (FAD) co-factor. First analyte reagent portion 106a can be applied using any suitable technique.

Second analyte reagent portion 106b can be any suitable mixture of reagents known to those of skill in the art that selectively reacts with a second analyte such as, for example the ketone 3-hydroxybutyrate, in a bodily fluid sample to form an electroactive species, which can then be quantitatively measured at the second working electrode of co-facial multi-analyte test strips according to embodiments of the present invention. Therefore, second analyte reagent portion 106b includes at least an enzyme and a mediator. Second analyte reagent portion 106b can be applied using any suitable technique. It should be noted that the first and second analytes are dissimilar. In other words, the first and second analytes are not the same chemical species. Therefore, two different analytes are determined by co-facial multi-analyte test strips according to the present invention.

When the second analyte is the ketone 3-hydroxybutyrate, the mediator can be, for example, a mixture of potassium ferricyanide and NAD and the enzyme can be, for example, a mixture of diaphorase and hydroxybutyrate dehydrogenase.

Once apprised of the present invention, one skilled in the art will recognize that first analyte reagent portion 106a and second analyte reagent portion 106b can, if desired, also contain suitable buffers (such as, for example, Tris HCl, Citraconate, Citrate and Phosphate), surfactants (for example, Tritoan X100, Tergitol NP-&, PLuronic F68, Betaine and Igepal), thickeners (including, for example, hydroxyethylcelulose, HEC, carboxymethylcellulose, ethycellulose and alginate) and other additives as are known in the field.

Test meters for use with a co-facial multi-analyte test strip according to embodiments of the present invention include a test strip receiving module and a signal processing module. The test strip receiving module has a first electrical connector configured for contacting a first analyte contact pad of a first working electrode of the co-facial multi-analyte test strip; a second electrical connector configured for contacting a counter/reference electrode contact pad of a shared counter/reference electrode layer of the co-facial multi-analyte test strip; and a third electrical connector configured for contacting a second analyte contact pad of a second working electrode of the co-facial multi-analyte test strip.

The signal processing module is configured to receive a first electrical signal via the first electrical connector and the second electrical connector and employ that first signal for the determination of a first analyte (such as glucose) in a bodily fluid sample applied to the co-facial multi-analyte test strip. The signal processing module is also configured to receive a second electrical signal via the second electrical connector and third electrical connector and employ the second electrical signal for the determination of a second analyte (such as the ketone 3-hydroxybutyrate) in the bodily fluid sample applied to the co-facial multi-analyte test strip. Furthermore, the first analyte electrode, and second analyte electrode are disposed on a first insulating layer of the co-facial multi-analyte test strip in a planar inline configuration while the shared counter/reference electrode layer is disposed in an opposing (co-facial) configuration with respect to the first and second working electrodes. The signal processing module can be configured to receive the first electrical signal and the second electrical signal sequentially, simultaneously or in a time-overlapping manner.

Test meters according to embodiments of the present invention are beneficially small in size, inexpensive and readily manufactured since they employ a single electrical connector for contacting a counter/reference electrode pad that is used for the determination of multiple analytes. In other words, only three electrical connectors are needed to determine multiple analytes, thus reducing test meter size, cost and manufacturing difficulty.

FIG. 4 is simplified depiction of a first electrically conductive layer 104 and shared counter/reference electrode layer 110 of a co-facial multi-analyte test strip according to an embodiment of the present invention in use with a test meter 200 also according to an embodiment of the present invention (with dashed line indicating features that are hidden from view in the perspective of FIG. 4). Test meter 200 includes a test strip receiving module 202 and a signal processing module 204 within case 206.

Test strip receiving module 202 includes a first electrical connector 208 configured for contacting first analyte contact pad 120 of a first working electrode of the co-facial multi-analyte test strip; a second electrical connector 210 configured for contacting a counter/reference electrode contact pad 128 of a shared counter/reference electrode layer of the co-facial multi-analyte test strip; and a third electrical connector 212 configured for contacting a second analyte contact pad 124 of a second working electrode of the co-facial multi-analyte test strip.

Signal processing module 204 is configured to receive a first signal via first electrical connector 208 and second electrical connector 210 and employ the first signal for the determination of a first analyte in a bodily fluid sample applied to the co-facial multi-analyte test strip. Signal processing module 204 is also configured to receive a second signal via second electrical connector 210 and third electrical connector 212 and employ the second signal for the determination of a second analyte in the bodily fluid sample applied to the co-facial multi-analyte test strip.

In the embodiment of FIG. 4, signal processing module 204 includes, for example, a signal receiving component, a signal measurement component, a processor component and a memory component (each not shown in FIG. 4). Test meter 200 can measure, for example, electrical resistance, electrical continuity or other electrical characteristic between first working electrode 118 and shared counter/reference electrode layer 110 and between second working electrode 122 and shared counter/reference electrode layer 110. One skilled in the art will appreciate that the test meter 200 can also employ a variety of sensors and circuits that are not depicted in simplified FIG. 4 during determination of a first analyte and a second analyte.

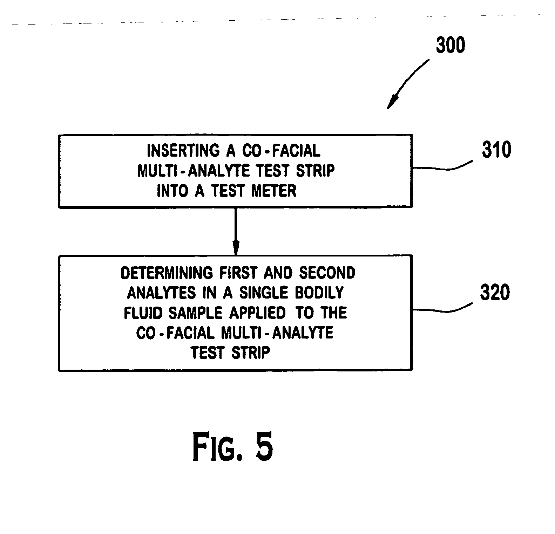

FIG. 5 is a flow diagram depicting stages in a method 300 for determining multiple analytes (for example, the analyte glucose and the analyte 3-hydroxybutyrate) in a single bodily fluid sample (such as a whole blood sample) according to an embodiment of the present invention.

At step 310 of method 300, a co-facial multi-analyte test strip is inserted into a test meter. The insertion of the test strip into the meter is such that a first electrical connector of the test meter comes into contact with a first analyte contact pad of a first working electrode of the co-facial multi-analyte test strip; a second electrical connector of the test meter comes into contact with a counter/reference electrode contact pad of a shared counter/reference electrode layer of the co-facial multi-analyte test strip; and a third electrical connector of the test meter comes into contact with a second analyte contact pad of a second working electrode of the co-facial multi-analyte test strip.

The method also includes determining at least a first analyte and a second analyte in a single bodily fluid sample applied to the multi-analyte test strip using a signal processing module of the test meter (see step 320 of FIG. 5). During the determining step, the signal processing module receives a first signal via the first electrical connector and the second electrical connector and employs the first signal for the determination of a first analyte. Also during the determining step, the signal processing module receives a second signal via the second electrical connector and the third electrical connector and employs the second signal for the determination of a second analyte.

Once apprised of the present disclosure, one skilled in the art will recognize that method 300 can be readily modified to incorporate any of the techniques, benefits and characteristics of co-facial multi-analyte test strips according to embodiments of the present invention and described herein, as well as those of test meters according to embodiments of the present invention described herein. Moreover, the bodily fluid sample can be applied to the co-facial multi-analyte test strip either before the inserting step or after the inserting step.

## Claims

1. A co-facial multi-analyte test strip comprising:
a first insulating layer;
an electrically conductive layer disposed on the first insulating layer, the electrically conductive layer including:
a first working electrode with a first analyte contact pad; and
a second working electrode with a second analyte contact pad;
a patterned spacer layer positioned above the electrically conductive layer, the patterned spacer layer defining a single bodily fluid sample-receiving chamber therein that overlies the first working electrode and the second working electrode, the single bodily fluid sample-receiving chamber having a proximal end and a distal end;
a shared counter/ reference electrode layer overlying and exposed to the sample receiving chamber, the shared counter/reference electrode layer configured in an opposing relationship to the first working electrode and the second working electrode, the shared counter/reference electrode layer having a counter/reference electrode contact pad; and
a second insulating layer disposed above the shared counter/reference electrode layer;
wherein the co-facial multi-analyte test strip further includes:
a multi-analyte reagent layer disposed on the electrically conductive layer, the multi-analyte reagent layer including:
a first analyte reagent portion disposed on at least a portion of the first working electrode within the sample receiving chamber; and
a second analyte reagent portion disposed on at least a portion of the second working electrode within the sample receiving chamber; and
wherein the first working electrode and second working electrode are disposed on the first insulating layer in a planar inline configuration.

2. The co-facial multi-analyte test strip of claim 1 further including a vent opening in fluidic communication with the distal end of the single bodily-fluid sample receiving chamber.

3. The co-facial multi-analyte test strip of claim 2 wherein the vent opening extends entirely through the first insulating layer, electrically conductive layer, patterned spacer layer, shared counter/reference electrode layer and second insulating layer.

4. The co-facial multi-analyte test strip of claim 2 wherein the vent opening is further configured as a stop flow junction at the distal end of the single bodily fluid sample-receiving chamber.

5. The co-facial multi-analyte test strip of claim 1 wherein the test strip has a test strip proximal end and a test strip distal end and the proximal end of the single bodily fluid sample-receiving chamber is open to the proximal end of the test strip..

6. The co-facial multi-analyte test strip of claim 1 wherein the first analyte reagent portion is nonidentical in comparison to the second analyte reagent portion.

7. The co-facial multi-analyte test strip of claim 5 wherein the first analyte reagent portion is a glucose analyte reagent.

8. The co-facial multi-analyte test strip of claim 6 wherein the second analyte reagent portion is a ketone analyte reagent.

9. The co-facial multi-analyte test strip of claim 8 wherein the ketone is 3-hydroxybutyrate.

10. The co-facial multi-analyte test strip of claim 1 wherein the bodily fluid sample is a whole blood sample.

11. A test meter for use with a co-facial multi-analyte test strip, the test meter comprising:
a test strip receiving module with at least:
a first electrical connector configured for contacting a first analyte contact pad of a first working electrode of the co-facial multi-analyte test strip;
a second electrical connector configured for contacting a counter/reference electrode contact pad of a shared counter/reference electrode of the multi-analyte test strip; and
a third electrical connector configured for contacting a second analyte contact pad of a second working electrode of the multi-analyte test strip; and
a signal processing module,
wherein the signal processing module is configured to receive a first signal via the first electrical connector and the second electrical connector and employ the first signal for the determination of a first analyte in a bodily fluid sample applied to the co-facial multi-analyte test strip; and
wherein the signal processing module is also configured to receive a second signal via the second electrical connector and third electrical connector and employ the second signal for the determination of a second analyte in the bodily fluid sample applied to the co-facial multi-analyte test strip; and
wherein the first electrical connector and third electrical connector are configured for essentially coplanar contact with the first analyte contact pad and the second analyte contact pad and the second electrical connector is configured to contact the counter/reference electrode contact pad in a manner offset from the essentially coplanar contact.

12. The test meter of claim 11 wherein the second electrical connector is disposed between the first electrical connector and the third electrical connector.

13. The test meter of claim 11 wherein the signal processing module is configured for determination of the first analyte and determination of the second analyte via an electrochemical-based determination technique.

14. The test meter of claim 11 wherein the signal processing module is configured to receive the first signal and the second signal in a sequential manner.

15. The test meter of claim 11 wherein the signal processing module is configured to receive the first signal and the second signal in a simultaneous manner.

16. A method for determining multiple analytes in a single bodily fluid sample, the method comprising:
inserting a co-facial multi-analyte test strip into a test meter such that:
a first electrical connector of the test meter comes into contact with a first analyte contact pad of a first working electrode of the multi-analyte test strip;
a second electrical connector of the test meter comes into contact with a counter/reference electrode contact pad of a shared counter/reference electrode of the multi-analyte test strip; and
a third electrical connector of the test meter comes into contact with a second analyte contact pad of a second working electrode of the multi-analyte test strip;
determining a first analyte and a second analyte in a single bodily fluid sample applied to the co-facial multi-analyte test strip using a signal processing module of the test meter,
wherein, during the determining step, the signal processing module receives a first signal via the first electrical connector and the second electrical connector and employs the first signal for the determination of the first analyte; and
wherein, during the determining step, the signal processing module receives a second signal via the second electrical connector and the third electrical connector and employs the second signal for the determination of the second analyte; and
wherein the first working electrode and second working electrode are disposed on a first insulating layer of the co-facial multi-analyte test strip in a planar inline configuration; and
wherein the shared counter/reference electrode layer is configured in an opposing relationship to the first working electrode and the second working electrode.

## Patentansprüche

1. Teststreifen für mehrere gegenüberliegende Analyten, der Folgendes umfasst:
eine erste Isolierschicht;
eine auf der ersten Isolierschicht angeordnete elektrisch leitende Schicht, wobei die elektrisch leitende Schicht Folgendes aufweist:
eine erste Arbeitselektrode mit einem Kontaktpad für einen ersten Analyten; und
eine zweite Arbeitselektrode mit einem Kontaktpad für einen zweiten Analyten;
eine gemusterte Abstandsschicht, die über der elektrisch leitenden Schicht angeordnet ist und eine einzelne Kammer zur Aufnahme einer Körperflüssigkeitsprobe darin definiert, die über der ersten Arbeitselektrode und der zweiten Arbeitselektrode liegt, wobei die einzelne Kammer zur Aufnahme einer Körperflüssigkeitsprobe ein proximales Ende und ein distales Ende aufweist;
eine gemeinsam genutzte Gegen/Referenzelektrodenschicht, die über der Probenaufnahmekammer liegt und dieser ausgesetzt ist, wobei die gemeinsam genutzte Gegen/Referenzelektrodenschicht in entgegengesetzter Beziehung zur ersten Arbeitselektrode und zur zweiten Arbeitselektrode konfiguriert ist, wobei die gemeinsam genutzte Gegen/Referenzelektrodenschicht ein Gegen/Referenzelektroden-Kontaktpad aufweist; und
eine zweite Isolierschicht, die auf der gemeinsam genutzten Gegen/Referenzelektrodenschicht angeordnet ist;
worin der zweiseitige Teststreifen für mehrere Analyten ferner Folgendes aufweist :
eine Reagenzschicht für mehrere Analyten, die auf der elektrisch leitenden Schicht angeordnet ist und Folgendes aufweist:
einen Reagenzabschnitt für einen ersten Analyten, der auf zumindest einem Teil der ersten Arbeitselektrode in der Probenaufnahmekammer angeordnet ist; und
einen Reagenzabschnitt für einen zweiten Analyten, der auf zumindest einem Teil der zweiten Arbeitselektrode in der Probenaufnahmekammer angeordnet ist; und
worin die erste Arbeitselektrode und die zweite Arbeitselektrode auf der ersten Isolierschicht in einer ebenen reihenförmigen Konfiguration angeordnet sind.

2. Teststreifen für mehrere gegenüberliegende Analyten nach Anspruch 1, der ferner eine Entlüftungsöffnung aufweist, die mit dem distalen Ende der einzelnen Kammer zur Aufnahme einer Körperflüssigkeit in Fluidikverbindung steht.

3. Teststreifen für mehrere gegenüberliegende Analyten nach Anspruch 2, worin sich die Entlüftungsöffnungen vollständig durch die erste Isolierschicht, die elektrisch leitende Schicht, die gemusterte Abstandsschicht, die gemeinsam genutzte Gegen/Referenzelektrodenschicht und die zweite Isolierschicht erstrecken.

4. Teststreifen für mehrere gegenüberliegende Analyten nach Anspruch 2, worin die Entlüftungsöffnung ferner als Strömungsunterbrechungsverbindung am distalen Ende der einzelnen Kammer zur Aufnahme einer Körperflüssigkeitsprobe konfiguriert ist.

5. Teststreifen für mehrere gegenüberliegende Analyten nach Anspruch 1, worin der Teststreifen ein proximales Teststreifenende und ein distales Teststreifenende aufweist und das proximale Ende der einzelnen Kammer zur Aufnahme einer Körperflüssigkeitsprobe zum proximalen Ende des Teststreifens hin offen ist.

6. Teststreifen für mehrere gegenüberliegende Analyten nach Anspruch 1, worin der Reagenzabschnitt für den ersten Analyten mit dem Reagenzabschnitt für den zweiten Analyten nicht identisch ist.

7. Teststreifen für mehrere gegenüberliegende Analyten nach Anspruch 5, worin der Reagenzabschnitt für den ersten Analyten ein Glukose-Analyten-Reagenz ist.

8. Teststreifen für mehrere gegenüberliegende Analyten nach Anspruch 6, worin der Reagenzabschnitt für den zweiten Analyten ein Keton-Analyten-Reagenz ist.

9. Teststreifen für mehrere gegenüberliegende Analyten nach Anspruch 8, worin das Keton 3-Hydroxybutyrat ist.

10. Teststreifen für mehrere gegenüberliegende Analyten nach Anspruch 1, worin die Körperflüssigkeitsprobe eine Vollblutprobe ist.

11. Testmessgerät zur Verwendung mit einem Teststreifen für mehrere gegenüberliegende Analyten, wobei das Testmessgerät Folgendes umfasst:
ein Teststreifen-Aufnahmemodul mit zumindest:
einem ersten elektrischen Konnektor für den Kontakt mit einem Kontaktpad für einen ersten Analyten einer ersten Arbeitselektrode des Teststreifens für mehrere gegenüberliegende Analyten;
einem zweiten elektrischen Konnektor für den Kontakt mit einem Gegen/Referenzelektroden-Kontaktpad einer gemeinsam genutzten Gegen/Referenzelektrode des Teststreifens für mehrere gegenüberliegende Analyten; und
einem dritten elektrischen Konnektor für den Kontakt mit einem Kontaktpad für einen zweiten Analyten einer zweiten Arbeitselektrode des Teststreifens für mehrere gegenüberliegende Analyten; und einem Signalverarbeitungsmodul,
worin das Signalverarbeitungsmodul so konfiguriert ist, dass es ein erstes Signal über den ersten elektrischen Konnektor und den zweiten elektrischen Konnektor empfangen kann und das erste Signal zur Bestimmung eines ersten Analyten in einer Körperflüssigkeitsprobe, die auf den Teststreifen für mehrere gegenüberliegende Analyten aufgebracht ist, verwenden kann; und
worin das Signalverarbeitungsmodul auch so konfiguriert ist, dass es ein zweites Signal über den zweiten elektrischen Konnektor und den dritten elektrischen Konnektor empfangen kann und das zweite Signal zur Bestimmung eines zweiten Analyten in der Körperflüssigkeitsprobe, die auf den Teststreifen für mehrere gegenüberliegende Analyten aufgebracht ist, verwenden kann; und
worin der erste elektrische Konnektor und der dritte elektrische Konnektor für im Wesentlichen planparallelen Kontakt mit dem Kontaktpad für den ersten Analyten und dem Kontaktpad für den zweiten Analyten konfiguriert sind und der zweite elektrische Konnektor für den Kontakt mit dem Gegen/Referenzelektroden-Kontaktpad in einer von dem im Wesentlichen planparallelen Kontakt versetzten Weise konfiguriert ist.

12. Testmessgerät nach Anspruch 11, worin der zweite elektrische Konnektor zwischen dem ersten elektrischen Konnektor und dem dritten elektrischen Konnektor angeordnet ist.

13. Testmessgerät nach Anspruch 11, worin das Signalverarbeitungsmodul zur Bestimmung des ersten Analyten und zur Bestimmung des zweiten Analyten mittels einer Bestimmungstechnik auf elektrochemischer Basis konfiguriert ist.

14. Testmessgerät nach Anspruch 11, worin das Signalverarbeitungsmodul zum sequentiellen Empfang des ersten Signals und des zweiten Signals konfiguriert ist.

15. Testmessgerät nach Anspruch 11, worin das Signalverarbeitungsmodul zum gleichzeitigen Empfang des ersten Signals und des zweiten Signals konfiguriert ist.

16. Verfahren zur Bestimmung mehrerer Analyten in einer einzelnen Körperflüssigkeitsprobe, wobei das Verfahren Folgendes umfasst:
Einführung eines Teststreifens für mehrere gegenüberliegende Analyten in ein Testmessgerät, so dass:
ein erster elektrischer Konnektor des Testmessgeräts mit einem Kontaktpad für einen ersten Analyten einer ersten Arbeitselektrode des Teststreifens für mehrere Analyten in Kontakt kommt;
ein zweiter elektrischer Konnektor des Testmessgeräts mit einem Gegen/Referenzelektroden-Kontaktpad einer gemeinsam genutzten Gegen/Referenzelektrode des Teststreifens für mehrere Analyten in Kontakt kommt; und
ein dritter elektrischer Konnektor des Testmessgeräts mit einem Kontaktpad für einen zweiten Analyten einer zweiten Arbeitselektrode des Teststreifens für mehrere Analyten in Kontakt kommt;
Bestimmung eines ersten Analyten und eines zweiten Analyten in einer einzelnen Körperflüssigkeitsprobe, die auf den Teststreifen für mehrere gegenüberliegende Analyten aufgebracht wurde, unter Verwendung eines Signalverarbeitungsmoduls des Testmessgeräts,
worin während des Bestimmungsschritts das Signalverarbeitungsmodul ein erstes Signal über den ersten elektrischen Konnektor und den zweiten elektrischen Konnektor empfängt und das erste Signal zur Bestimmung des ersten Analyten verwendet; und
worin während des Bestimmungsschritts das Signalverarbeitungsmodul ein zweites Signal über den zweiten elektrischen Konnektor und den dritten elektrischen Konnektor empfängt und das zweite Signal zur Bestimmung des zweiten Analyten verwendet; und worin die erste Arbeitselektrode und die zweite Arbeitselektrode auf einer ersten Isolierschicht des Teststreifens für mehrere gegenüberliegende Analyten in ebener reihenförmiger Konfiguration angeordnet sind; und
worin die gemeinsam genutzte Gegen/Referenzelektrodenschicht in entgegengesetzter Beziehung zur ersten Arbeitselektrode und zur zweiten Arbeitselektrode konfiguriert ist.

## Revendications

1. Bandelette réactive multianalyte cofaciale comprenant :
une première couche isolante ;
une couche électriquement conductrice disposée sur la première couche isolante, la couche électriquement conductrice comportant :
une première électrode de travail avec un premier patin de contact avec les analytes ; et
une deuxième électrode de travail avec un deuxième patin de contact avec les analytes ;
une couche d'espacement façonnée positionnée au-dessus de la couche électriquement conductrice, la couche d'espacement façonnée définissant une chambre de réception d'un seul échantillon de liquide biologique qui recouvre la première électrode de travail et la deuxième électrode de travail, la chambre de réception d'un seul échantillon de liquide biologique ayant une extrémité proximale et une extrémité distale ;
une couche de contre-électrode/électrode de référence partagée recouvrant et exposée à la chambre de réception d'échantillon, la couche de contre-électrode/électrode de référence partagée étant configurée dans une relation d'opposition par rapport à la première électrode de travail et la deuxième électrode de travail, la couche de contre-électrode/électrode de référence partagée comportant un patin de contact de contre-électrode/électrode de référence ; et
une deuxième couche isolante disposée au-dessus de la couche de contre-électrode/électrode de référence partagée ;
la bandelette réactive multianalyte cofaciale comprenant en outre :
une couche de réactif multianalyte disposée sur la couche électriquement conductrice, la couche de réactif multianalyte comportant :
une première partie de réactif d'analyte disposée sur au moins une partie de la première électrode de travail à l'intérieur de la chambre de réception d'échantillon ; et
une deuxième partie de réactif d'analyte disposée sur au moins une partie de la deuxième électrode de travail à l'intérieur de la chambre de réception d'échantillon ; et
dans laquelle la première électrode de travail et la deuxième électrode de travail sont disposées sur la première couche isolante dans une configuration planaire en ligne.

2. Bandelette réactive multianalyte cofaciale selon la revendication 1 comprenant en outre un orifice de ventilation en communication fluidique avec l'extrémité distale de la chambre de réception d'un seul échantillon de liquide biologique.

3. Bandelette réactive multianalyte cofaciale selon la revendication 2 dans laquelle l'orifice de ventilation s'étend à travers la totalité de la première couche isolante, la couche électriquement conductrice, la couche d'espacement façonnée, la couche de contre-électrode/électrode de référence partagée et la deuxième couche isolante.

4. Bandelette réactive multianalyte cofaciale selon la revendication 2 dans laquelle l'orifice de ventilation est en outre configuré comme une jonction d'arrêt d'écoulement à l'extrémité distale de la chambre de réception d'un seul échantillon de liquide biologique.

5. Bandelette réactive multianalyte cofaciale selon la revendication 1, la bandelette réactive ayant une extrémité proximale de bandelette réactive et une extrémité distale de bandelette réactive, et l'extrémité proximale de la chambre de réception d'un seul échantillon de liquide biologique étant ouverte sur l'extrémité proximale de la bandelette réactive.

6. Bandelette réactive multianalyte cofaciale selon la revendication 1 dans laquelle la première partie de réactif d'analyte n'est pas identique à la deuxième partie de réactif d'analyte.

7. Bandelette réactive multianalyte cofaciale selon la revendication 5 dans laquelle la première partie de réactif d'analyte est un réactif de l'analyte glucose.

8. Bandelette réactive multianalyte cofaciale selon la revendication 6 dans laquelle la deuxième partie de réactif d'analyte est un réactif de l'analyte cétone.

9. Bandelette réactive multianalyte cofaciale selon la revendication 8 dans laquelle la cétone est le 3-hydroxybutyrate.

10. Bandelette réactive multianalyte cofaciale selon la revendication 1 dans laquelle l'échantillon de liquide biologique est un échantillon de sang total.

11. Contrôleur à utiliser avec une bandelette réactive multianalyte cofaciale, le contrôleur comprenant :
un module de réception de bandelette réactive avec au moins :
un premier connecteur électrique configuré pour entrer en contact avec un premier patin de contact avec les analytes d'une première électrode de travail de la bandelette réactive multianalyte cofaciale ;
un deuxième connecteur électrique configuré pour entrer en contact avec un patin de contact de contre-électrode/électrode de référence de la bandelette réactive multianalyte ; et
un troisième connecteur électrique configuré pour entrer en contact avec un deuxième patin de contact avec les analytes d'une deuxième électrode de travail de la bandelette réactive multianalyte ; et
un module de traitement du signal,
dans lequel le module de traitement du signal est configuré pour recevoir un premier signal par l'intermédiaire du premier connecteur électrique et du deuxième connecteur électrique et utiliser le premier signal pour la détermination d'un premier analyte dans un échantillon de liquide biologique appliqué à la bandelette réactive multianalyte cofaciale ;
dans lequel le module de traitement du signal est également configuré pour recevoir un deuxième signal par l'intermédiaire du deuxième connecteur électrique et du troisième connecteur électrique et utiliser le deuxième signal pour la détermination d'un deuxième analyte dans l'échantillon de liquide biologique appliqué à la bandelette réactive multianalyte cofaciale ; et
dans lequel le premier connecteur électrique et le troisième connecteur électrique sont configurés pour un contact essentiellement coplanaire avec le premier patin de contact avec les analytes et le deuxième patin de contact avec les analytes, et le deuxième connecteur électrique est configuré pour entrer en contact avec le patin de contact de contre-électrode/électrode de référence de manière décalée par rapport au contact essentiellement coplanaire.

12. Contrôleur selon la revendication 11 dans lequel le deuxième connecteur électrique est disposé entre le premier connecteur électrique et le troisième connecteur électrique.

13. Contrôleur selon la revendication 11 dans lequel le module de traitement du signal est configuré pour la détermination du premier analyte et la détermination du deuxième analyte par une technique de détermination électrochimique.

14. Contrôleur selon la revendication 11 dans lequel le module de traitement du signal est configuré pour recevoir le premier signal et le deuxième signal de manière séquentielle.

15. Contrôleur selon la revendication 11 dans lequel le module de traitement du signal est configuré pour recevoir le premier signal et le deuxième signal de manière simultanée.

16. Procédé pour déterminer de multiples analytes dans un seul échantillon de liquide biologique, le procédé comprenant :
l'insertion d'une bandelette réactive multianalyte cofaciale dans un contrôleur de telle sorte que :
un premier connecteur électrique du contrôleur vient en contact avec un premier patin de contact avec les analytes d'une première électrode de travail de la bandelette réactive multianalyte ;
un deuxième connecteur électrique du contrôleur vient en contact avec un patin de contact de contre-électrode/électrode de référence de la bandelette réactive multianalyte ; et
un troisième connecteur électrique du contrôleur vient en contact avec un deuxième patin de contact avec les analytes d'une deuxième électrode de travail de la bandelette réactive multianalyte ; et
la détermination d'un premier analyte et d'un deuxième analyte dans un seul échantillon de liquide biologique appliqué à la bandelette réactive multianalyte cofaciale en utilisant un module de traitement du signal du contrôleur,
dans lequel, lors de l'étape de détermination, le module de traitement du signal reçoit un premier signal par l'intermédiaire du premier connecteur électrique et du deuxième connecteur électrique et utilise le premier signal pour la détermination du premier analyte ;
dans lequel, lors de l'étape de détermination, le module de traitement du signal reçoit un deuxième signal par l'intermédiaire du deuxième connecteur électrique et du troisième connecteur électrique et utilise le deuxième signal pour la détermination du deuxième analyte ;
dans lequel la première électrode de travail et la deuxième électrode de travail sont disposées sur une première couche isolante de la bandelette réactive multianalyte cofaciale dans une configuration planaire en ligne ; et
dans lequel la couche de contre-électrode/électrode de référence partagée est configurée dans une relation d'opposition par rapport à la première électrode de travail et la deuxième électrode de travail.
